# EUROPEAN PATENT APPLICATION

(11) **EP 4 616 850 A1**
(43) Date of publication of application: **17.09.2025**
(21) Application number: 23889193.1
(22) Date of filing: 10.11.2023
(51) Int. Cl.: A61K 9/20, A61K 9/28, A61K 31/454, A61P 43/00

(54) **CONTROLLED RELEASE PHARMACEUTICAL COMPOSITION**

(30) Priority: 11.11.2022 KR 20220150695
(71) Applicant: Daewoong Pharmaceutical Co., Ltd., Hwaseong-si, Gyenoggi-do 18623 (KR)
(72) Inventor: KWON, Seonju, Yongin-si, Gyeonggi-do 17000 (KR); HA, Songyi, Yongin-si, Gyeonggi-do 16863 (KR); KIM, Gyoung Won, Yongin-si, Gyeonggi-do 16995 (KR); HWANG, On, Yongin-si, Gyeonggi-do 16863 (KR); KIM, Gwan Young, Yongin-si, Gyeonggi-do 07292 (KR); CHO, Sang Eun, Yongin-si, Gyeonggi-do 16899 (KR); KIM, Dongyoon, Seoul 08655 (KR); PARK, Min Young, Suwon-si, Gyeonggi-do 16513 (KR); CHOI, Jongwon, Seoul 08096 (KR)
(74) Representative: Germain Maureau
(86) International application number: PCT/KR2023/018035
(87) International publication number: WO 2024/101937

(57) **Abstract**

The present invention relates to a formulation that can inhibit the initial release of a specific PRS inhibitor, and has the effect of preventing side effects of nausea or vomiting that occur when an excessively large amount of the active ingredient is absorbed in the initial stage.

## Description

### [TECHNICAL FIELD]

The present invention relates to a pharmaceutical composition comprising a specific PRS inhibitor, and more particularly to an initial controlled release pharmaceutical composition.

### [BACKGROUND ART]

PRS (prolyl-tRNA synthetase) is one of the aminoacyl-tRNA synthetase (ARS) family and serves to activate an amino acid for protein synthesis. That is, ARS performs a translational function to form aminoacyl adenylate (AA-AMP) and then transfer the activated amino acid to the 3-end of the corresponding tRNA. Since ARS plays an important role in the synthesis of protein, the inhibition of ARS inhibits the growth of all cells. Thus, ARS has been recognized as a promising target for a therapeutic agent for treating diseases that should inhibit antibiotics or cell overexpression (Nature 2013, 494:121-125).

PRS is present in, or functions as, a multisynthetase complex (MSC) in the form of EPRS (Glutamyl-Prolyl-tRNA Synthetase). In particular, among various MSCs, EPRS functions as a translational silencer that suppresses the production of VEGF (vascular endothelial growth factor A) which is a key factor in angiogenesis. In addition, it is reported that EPRS is closely related with various solid tumors (Nat. Rev. Cancer, 2011, 11, 708-718).

On the other hand, Korean Patent No. 10-2084772 discloses a PRS inhibitor having the following structure, which is a substance that is attracting attention as a preventive or therapeutic agent for PRS-related diseases, especially fibrosis.

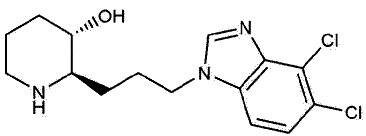

However, as a result of various clinical testing on the above substance, it was confirmed that there were side effects of nausea or vomiting. Therefore, the present inventors have conducted intensive research on methods to improve these side effects, and as a result, found that this problem can be solved by preparing a formulation containing the above substances so as to meet specific conditions, and completed the present invention.

### [DETAILED DESCRIPTION OF THE INVENTION]

### [Technical Problem]

It is an object of the present invention to provide a pharmaceutical composition comprising a specific PRS inhibitor that can control the initial release.

### [Technical Solution]

In order to achieve the above object, according to the present invention, there is provided a controlled release pharmaceutical composition comprising: a compound represented by the following Chemical Formula 1, or a pharmaceutically acceptable salt thereof; a binder; and one or more pharmaceutically acceptable excipients, wherein the pharmaceutical composition shows a dissolution rate of 75% or less within 5 minutes in a pH 6.8 solution at 37°C based on an uncoated tablet:

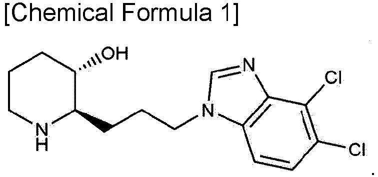

The compound represented by Chemical Formula 1 or a pharmaceutically acceptable salt thereof is a compound described in Korean Patent No. 10-2084772, and is specifically a material described in Example 40 of that specification. The above material is expected to be used as a PRS inhibitor to prevent or treat fibrosis, but as a result of various clinical testing, there was a problem that drug resistance is poor due to side effects such as nausea or vomiting. Therefore, in order to improve drug compliance, it is necessary to improve these side effects. After examining the causes of the above side effects from various perspectives, it has been found that it is a side effect caused by irritation of the gastrointestinal tract. In particular, it was confirmed that the possibility of side effects is high when an excessively large amount of drug is absorbed in the initial stage.

Therefore, the present inventors confirmed that when the initial release rate is controlled to be low in the preparation of a formulation containing the above material, the side effects that occur when an excessively larger drug is absorbed in the initial stage can be significantly reduced.

In particular, in a formulation containing the compound represented by Chemical Formula 1, or a pharmaceutically acceptable salt thereof (hereinafter referred to as 'active ingredient'), the present invention is characterized by providing a controlled-release pharmaceutical composition that shows a dissolution rate of 75% or less within 5 minutes in a pH 6.8 solution at 37°C based on an uncoated tablet. Particularly, it is characterized in that the active ingredient and the ingredients contained in the formulation are physically bound together using the binder to control initial release.

Preferably, the controlled release pharmaceutical composition according to the present invention shows a dissolution rate of 65% or less within 5 minutes in a pH 6.8 solution at 37°C based on uncoated tablets. Preferably, the controlled release pharmaceutical composition according to the present invention shows a dissolution rate of 90% or more within 30 minutes in a pH 6.8 solution at 37°C based on an uncoated tablet. Preferably, the controlled release pharmaceutical composition according to the present invention does not elute in a pH 1.2 solution at 37°C based on an uncoated tablet. In other words, the controlled release pharmaceutical composition according to the present invention is acid-resistant and has the characteristic that the initial dissolution rate is inhibited, but all of the drug is released after a certain period of time.

Furthermore, the controlled release pharmaceutical composition according to the present invention may include a coating agent, and specifically, it may include a coating layer formed with a coating agent on the uncoated tablet. Preferably, the controlled release pharmaceutical composition according to the present invention is a coated tablet containing a coating agent, and shows a dissolution rate of 20% or less within 10 minutes in a pH 6.8 solution at 37°C. Preferably, the controlled release pharmaceutical composition according to the present invention shows a dissolution rate of less than 60% within 15 minutes in a pH 6.8 solution at 37°C based on the coated tablet. Preferably, the controlled release pharmaceutical composition according to the present invention shows a dissolution rate of 90% or more within 30 minutes in a pH 6.8 solution at 37°C based on the coated tablet. Preferably, the controlled release pharmaceutical composition according to the present invention does not elute in a pH 1.2 solution at 37°C based on the coated tablet. In other words, the controlled release pharmaceutical composition according to the present invention is acid-resistant and has the characteristic that the initial dissolution rate is inhibited, but all of the drug is released after a certain period of time. Preferably, the coated tablet is an enteric coated tablet. Further, preferably, the coating agent is contained in an amount of 6 to 12 parts by weight relative to 100 parts by weight of the uncoated tablet.

On the other hand, the amount of the binder used can be adjusted to thereby adjust the initial release rate of the controlled release pharmaceutical composition according to the present invention. From this point of view, preferably, the binder is contained in an amount of 1 to 20 parts by weight relative to 100 parts by weight of the uncoated tablet. More preferably, the binder is contained in an amount of 1.5 parts by weight or more, or 2.0 parts by weight or more, and 19 parts by weight or less, 18 parts by weight or less, 17 parts by weight or less, 16 parts by weight or less, 15 parts by weight or less, 14 parts by weight or less, 13 parts by weight or less, 12 parts by weight or less, 11 parts by weight or less, or 10 parts by weight or less relative to 100 parts by weight of the uncoated tablet.

The binder is not particularly limited as long as it can show the initial release rate as described above. Preferably polyvinylpyrrolidone, hydroxypropyl cellulose, carboxymethylcellulose sodium, carboxymethylcellulose calcium, hydroxyethyl cellulose, hydroxypropyl methylcellulose, gelatin, gum arabic, or xanthan gum can be used.

Preferably, the viscosity of the binder is 5 to 1000 mPa·s in a 10% *w*/*v* aqueous solution at 20°C. If the viscosity of the binder is less than 5 mPa·s in a 10% *w*/*v* aqueous solution at 20°C, the viscosity is too low and the role of the binder is insufficient, thereby making it difficult to control the initial release. If the viscosity of the binder exceeds 1000 mPa·s, the viscosity is too high and an excessively long time is required for the active ingredient to be released. Preferably, the viscosity of the binder is 10 mPa·s or more, 50 mPa·s or more, 100 mPa·s or more, 150 mPa·s or more, 200 mPa·s or more, 250 mPa·s or more, or 300 mPa·s or more, and 950 mPa·s or less, 900 mPa·s or less, 850 mPa·s or less, 800 mPa·s or less, 750 mPa·s or less, or 700 mPa·s or less in a 10% *w*/*v* aqueous solution at 20°C. Preferably, the binder is polyvinylpyrrolidone, and the above-mentioned viscosity can be applied.

Preferably, the viscosity of the binder is 5 to 500 mPa·s in a 2% *w*/*v* aqueous solution at 20°C. In the same manner as above, if the viscosity of the binder is less than 5 mPa·s in a 2% *w*/*v* aqueous solution at 20°C, the viscosity is so low and the role of the binder is insufficient, thereby making it difficult to control the initial release. If the viscosity of the binder exceeds 500 mPa·s, the viscosity is too high and an excessively long time is required for the active ingredient to be released. Preferably, the viscosity of the binder is 5.5 mPa·s or more, or 6 mPa·s or more, and 400 mPa·s or less, 300 mPa·s or less, 200 mPa·s or less, 100 mPa·s or less, 50 mPa·s or less, 40 mPa·s or less, 30 mPa·s or less, 20 mPa·s or less, or 10 mPa·s or less in a 2% *w*/*v* aqueous solution at 20°C. Preferably, the binder is hydroxypropyl cellulose, and the above-mentioned viscosity can be applied.

The one or more pharmaceutically acceptable additives are not particularly limited as long as the above-mentioned initial release control effect is maintained. By way of example, the additives may include a disintegrant, an excipient, a lubricant, a diluent, and the like.

Among the above additives, the disintegrant plays the opposite role to that of a binder, and therefore, it is preferable not to use the disintegrant. Alternatively, when a disintegrant is used, it should be used within a range that does not inhibit the initial release effect resulting from the use of the binder. For example, a disintegrant with a low disintegration effect is used, or a disintegrant is used in as an amount as small as possible. From this point of view, preferably, the controlled release pharmaceutical composition according to the present invention contains 0 to 10 parts by weight of the disintegrant relative to 100 parts by weight of the uncoated tablet. That is, it does not contain a disintegrant, or contains 10 parts by weight or less of the disintegrant relative to 100 parts by weight of the uncoated tablet. Further, if the disintegrant is a super disintegrant with a high disintegration effect, the disintegrant is contained in an amount of 50 parts by weight or less relative to 100 parts by weight of the binder. That is, the composition does not contain a super disintegrant, or contains 50 parts by weight or less of the super disintegrant relative to 100 parts by weight of the binder.

Among the above additives, the excipient is not particularly limited as long as it is used in the preparation of the pharmaceutical composition. Although it is not closely related to controlling the release of drugs, it may affect the uniformity of the formulation, which indicates the degree to which the main ingredient content is uniform during the preparation of a pharmaceutical composition. Preferably, the excipient is contained in an amount of 100 to 200 parts by weight relative to 100 parts by weight of the compound represented by Chemical Formula 1, or a pharmaceutically acceptable salt thereof. More preferably, the excipient is contained in an amount of 110 parts by weight or more, 120 parts by weight or more, or 130 parts by weight or more, and 190 parts by weight or less, 180 parts by weight or less, 170 parts by weight or less, or 160 parts by weight or less relative to 100 parts by weight of the compound represented by Chemical Formula 1, or a pharmaceutically acceptable salt thereof.

On the other hand, the pharmaceutical composition according to the present invention may comprise (i) a pre-mixed granule containing a compound represented by Chemical Formula 1, or a pharmaceutically acceptable salt thereof; a binder; and an excipient, and (ii) a post-mixed material containing an excipient. As described below, the pre-mixed granule can be first prepared and then tableted together with the post-mixed material to prepare a pharmaceutical composition. At this time, the weight ratio of the excipient contained in the pre-mixed granule and the post-mixed material may have a certain influence on the uniformity of the formulation. From this point of view, in order to make secure formulation uniformity in the present invention, the weight ratio of the excipients contained in the pre-mixed granule and the post-mixed material is preferably 4:6 to 6:4.

On the other hand, the pharmaceutical composition according to the present invention includes mixing the compound represented by Chemical Formula 1, or a pharmaceutically acceptable salt thereof; a binder; and one or more pharmaceutically acceptable additives, and compression molding the prepared mixture.

More specifically, the pharmaceutical composition according to the present invention can be prepared by a preparation method comprising a step of mixing ingredients including a compound represented by Chemical Formula 1, or a pharmaceutically acceptable salt thereof; a binder; and an excipient to prepare a pre-mixed granule, and a step of mixing the pre-mixed granule and the post-mixed material containing an excipient and then compression molding the mixture.

### [Advantageous Effects]

As mentioned above, the present invention prepares a formulation capable of inhibiting the initial release of the active ingredient, and has the effect of preventing the side effects of nausea or vomiting that occur when an excessively larger amount of the drug is absorbed in the initial stage.

### [BRIEF DESCRIPTION OF THE DRAWINGS]

FIG. 1 shows the results of Experimental Example 1 of the present invention.
FIGS. 2 and 3 show the results of Experimental Example 2 of the present invention.

### [DETAILED DESCRIPTION OF THE EMBODIMENTS]

Hereinafter, the present invention will be explained in more detail with reference to the following examples. However, the following examples are for illustrative purposed only, and are not intended to limit the scope of the present invention in any way.

### Preparation Example

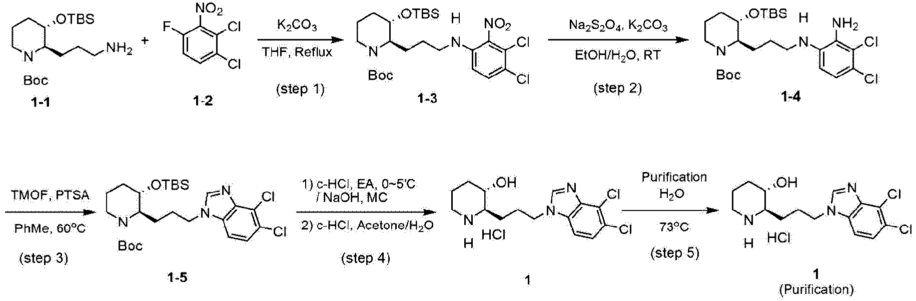

### Step 1) Preparation of Compounds 1-3

Tert-butyl (2R,3S)-2-(3-aminopropyl)-3-((tertbutyldimethylsilyl)oxy)piperidine-1-carboxylate (100.0 g, 0.27 mol, 1.0 eq), which is Compound 1-1, was dissolved in 1000 mL of tetrahydrofuran, to which 1,2-dichloro-4-fluoro-3-nitrobenzene (56.4 g, 0.27 mol, 1.0 eq), which is Compound 1-2, and potassium carbonate (K₂CO₃, 74.2g, 0.54 mol, 2.0 eq) were added, and the mixture was stirred for 1 to 3 hours under tetrahydrofuran reflux conditions (80 to 100°C). When the reaction was completed, 1000 mL of purified water was added to extract the organic layer, and then 1000 mL of ethyl acetate was added and re-extracted. After completion of reduced pressure concentration, 100 mL of ethanol was added to the concentrated residue, and the mixture was re-concentrated. 400 mL of ethanol was added to the concentrated residue, dissolved, and then crystallized. After crystals were formed, 400 mL of purified water was added, and then crystallized at 0-5°C for 2 hours. It was filtered under reduced pressure using a filter, and washed with a mixture of 100 mL of EtOH and 100 mL of purified water cooled to 0 to 5°C. This was vacuum dried at 45-55°C for 12 hours to obtain Compounds 1-3 as an orange or red solid, tert-butyl (2R,3S)-3-((tert-butyldimethylsilyl)oxy)-2-(3-((3,4-dichloro-2-nitro-phenyl)amino)propyl)piperidine-1-carboxylate (143.0 g, yield: 95%).

### Step 2) Preparation of Compounds 1-4

Compound 1-3 (120.0 g, 0.21 mol, 1.0 eq) obtained in step 1 was dissolved in 1200 mL of ethanol, then potassium carbonate (176.9 g, 1.28 mol, 6.0 eq) was added, 1400 mL of sodium hydrosulfite aqueous solution (content of Na₂S₂O₄ in aqueous solution: 222.8 g, 1.28 mol, 6.0 eq) was added, and then the mixture was stirred at room temperature for 1 hour. When the reaction was completed, ethanol was concentrated under reduced pressure, and then 600 mL of purified water and 1,200 mL of ethyl acetate were added and extracted. 600 mL of ethyl acetate was added and re-extracted. 1200 mL of brine was added to wash the organic layer. Sodium sulfate was added for drying moisture, and surplus moisture was removed. This was concentrated under reduced pressure to obtain Compound 1-4 as a brown liquid, tert-butyl (2R,3S)-2-(3-((2-amino-3,4-dichlorophenyl)amino)propyl)-3-((tertbutyldimethylsilyl)oxy)piperidine-1- carboxylate (113.6 g, yield: 100%). The obtained compound was used in the next step without purification.

### Step 3) Preparation of Compounds 1-5

Compound 1-4 (113.6 g, 0.21 mol, 1.0 eq) obtained in step 2 was dissolved in 1136 mL of toluene, and then trimethyl orthoformate (TMOF, 30.3 mL, 0.28 mol, 1.3eq) and paratoluenesulfonic acid (0.4 g, 0.02 mol, 0.1 eq) was added, and the mixture was stirred at 50-60°C for 1-2 hours. When the reaction was completed, 1136 mL of toluene used in the reaction was removed by concentration under reduced pressure. 122 mL of sodium bicarbonate aqueous solution, 1136 mL of ethyl acetate, and 1136 mL of purified water were added and extracted. At this time, layer separation was easily performed. Then, 568 mL of EA was added to the aqueous layer and re-extracted. To color the organic layer, activated carbon (11.4 g, 0.1 eq) was added and stirred for 15 minutes. Sodium sulfate was added to remove moisture, stirred for 15 minutes, and then stirred through celite. After the concentration of the filtrate under reduced pressure was completed, 227 mL of normal hexane was added to the concentrated residue, concentrated under reduced pressure. 340 mL of normal hexane was added thereto, and the mixture was refluxed and stirred for 30 minutes to loosen crystals, and then cooled to 0-5°C and stirred at the same temperature for 4 hours. It was filtered under reduced pressure using a filter, and washed with 113 mL of normal hexane cooled to 0-5°C. It was vacuum dried at 45-55°C for 12 hours to obtain Compounds 1-5 as a white solid, tert-butyl (2R, 3S)-3-((tert-butyldimethylsilyl)oxy)-2-(3-(4,5-dichloro-1H-benzo[d]imidazol-1-yl)propyl)piperidine-1-carboxylate (100 g, yield: 86%). Further, the filtrate was concentrated and the crystallization process was carried out in the same manner to further obtain the above Compound 1-5 (8.0 g, yield: 8%) as a white solid. The final Compound 1-5 (108 g, yield: 94%) was obtained.

¹H NMR (500 MHz, MeOD): δ 8.30 (s, 1H), 7.56 (d, 1H), 7.43 (d, 1H), 4.30 (m, 2H), 4.17 (s, 1H), 4.05 (s, 1H), 3.91 (d, 1H), 3.73 (s, 1H), 2.68 (s, 1H), 1.87 (s, 3H), 1.70 (t, 2H), 1.55 (d, 1H), 1.45 (m, 10H), 1.42 (s, 1H), 0.90 (s, 9H), 0.07 (d, 6H)

### Step 4) Preparation of the compound represented by Chemical Formula 1

Compound 1-5 (90.0 g, 0.17 mol, 1.0 eq) obtained in step 3 was dissolved in 540 mL of ethyl acetate, and cooled to 0-10°C. Concentrated hydrochloric acid (146.4 mL, 10.0 eq) was added, and stirred for 1 to 2 hours. After completion of the reaction, 540 mL of purified water was added, and extracted at room temperature (product exists in the aqueous layer under acidic conditions). 540 mL of ethyl acetate was added again to the aqueous layer, and re-extracted (impurities were removed with EA). The organic layer was discarded, and 8N aqueous sodium hydroxide solution was slowly added to adjust the pH to 12.5 or higher. 900 mL of dichloromethane was added thereto, and extracted (product exists in MC layer under basic conditions). 450 mL of dichloromethane was added and re-extracted. After completion of concentration under reduced pressure, the concentrated residue, freebase (salt-free) (2R,3S)-2-(3-(4,5-dichloro-1H-benzo[d]imidazol-1-yl)propyl)piperidine-3-ol was added to 1100 mL of acetone and 54 mL of purified water and dissolved. 1 Equivalent of concentrated hydrochloric acid was added dropwise in 3 portions, and the resulting crystals were stirred at 0-5°C for 4 hours, and crystallized. It was filtered under reduced pressure using a filter and washed with 109 mL of acetone cooled to 0-5°C. It was vacuum dried at 45-55°C for 12 hours to obtain a compound represented by Chemical Formula 1 as a white solid, (2R,3S)-2-(3-(4,5-dichloro-1H-benzo[d]imidazol-1-yl)propyl)piperidin-3-ol 1HCl (58.5 g, yield: 97%).

¹H NMR (500 MHz, DMSO): δ 8.45 (s, 1H), 7.71 (d, 1H), 7.47 (d, 1H), 5.40 (d, 1H), 4.32 (m, 2H), 3.41 (m, 1H), 3.08 (d, 1H), 2.75 (m, 2H), 2.07 (m, 1H), 1.97 (m, 1H), 1.85 (m, 2H), 1.75 (m, 1H), 1.65 (m, 1H), 1.52 (m, 1H), 1.35 (m, 1H)

### Step 5) Further purification step

110 mL of purified water was added to the compound represented by Chemical Formula 1 (55 g, 0.15 mol, 1.0 eq) obtained in step 4, and the mixture was stirred at 70-75°C for 1 hour, then cooled to 0-5°C and stirred at the same temperature for 4 hours. It was filtered under reduced pressure using a filter, and washed with 55 mL of acetone cooled to 0-5°C. It was vacuum dried at 45-55°C for 12 hours to obtain the compound represented by Chemical Formula 1 (52.0 g, yield: 94%) as a purified white solid. In the following, the compound represented by Chemical Formula 1 was referred to as 'API' or 'active ingredient'.

¹H NMR (500 MHz, DMSO): δ 8.45 (s, 1H), 7.71 (d, 1H), 7.47 (d, 1H), 5.40 (d, 1H), 4.32 (m, 2H), 3.41 (m, 1H), 3.08 (d, 1H), 2.75 (m, 2H), 2.07 (m, 1H), 1.97 (m, 1H), 1.85 (m, 2H), 1.75 (m, 1H), 1.65 (m, 1H), 1.52 (m, 1H), 1.35 (m, 1H)

### Experimental Example 1

In order to confirm the safety/tolerability of the active ingredient in the human body, side effects were checked in healthy adult subjects.

The clinical testing was designed for 32 healthy adult subjects in a randomized, double-blinded, placebo-controlled, single-dose escalating manner, as shown in Table 1 below. The capsule tablets used in this clinical testing were produced by using Vcaps^{®} enteric-coated capsules containing only the active ingredient in hydrochloride form without additional excipients, and filling capsules by volume. The ingredients of the enteric-coated tablet (1 tablet) used in the clinical testing are shown in Table 2 below, and were named 'Comparative Example 1'. In addition, for the enteric-coated tablet administrated group, 5 tablets were administered at a time.

**[Table 1]**

| Category | Group | Active ingredient | Formulation | Administration method |
|---|---|---|---|---|
| Single administratioin | SAD1 | 100 mg | Enteric coated capsule | Single oral administration in a hungry state |
| | SAD2 | 300 mg | | |
| | SAD3 | 600 mg | | |
| | SAD4 | 500 mg | Enteric coated tablet | |

**[Table 2]**

| Category | | Ingredient | Content(mg) |
|---|---|---|---|
| Premixing | Main ingredient | API | 111.11 |
| | Excipient | MCC(JRS-101) | 80.00 |
| | | Supertab 30GR | 80.00 |
| | Disintegrant | Crospovidone | 10.00 |
| | Lubricant | Mg Stearate | 3.00 |
| Post-mixing | Excipient | MCC(JRS-101) | 56.95 |
| | | Supertab 30GR | 56.95 |
| | Disintegrant | Crospovidone | 10.00 |
| | Binder | HPC-L | 10.00 |
| | Lubricant | Mg Stearate | 2.00 |
| Uncoated table | | | 420.00 |
| Coating | First coating agent | Opadry white 03B28796 | 8.00 |
| | Second coating agent | Acryl EZE 93O18508 | 34.00 |
| Coated tablet | | | 462.00 |

As a result of confirming the safety and drug resistance of a single administration, the most frequently occurring adverse reactions were gastrointestinal adverse reactions including nausea, vomiting, diarrhea, and abdominal pain. Of these, nausea and vomiting are evaluated to have a greater impact on safety and drug resistance, and it was confirmed that this was caused by the active ingredient itself.

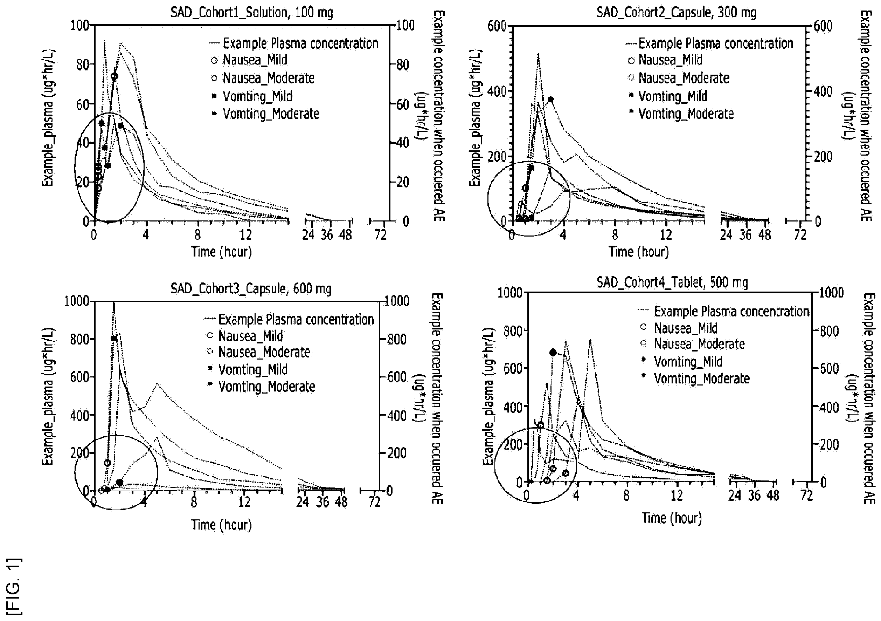

The relationship between the time of occurrence of these nausea and vomiting side effects and the exposure in the body was confirmed as shown in FIG. 1, and as a result, it has been confirmed that the majority of cases occur before the blood concentration in the body increases. This is understood that nausea and vomiting that occur upon administration of the active ingredient act on the gastrointestinal tract, rather than on the vomiting center activation pathway through chemical receptor-induced site stimulation due to exposure to blood in the body, thereby exciting the vagus nerve and activating the vomiting center.

### Experimental Example 2

Separately from the clinical testing in Experimental Example 1, the clinical testing was designed for 32 healthy adult subjects in a randomized, double-blinded, placebo-controlled, multi-dose escalating manner, as shown in Table 3 below. For MAD1 and MAD2 administrated groups, one tablet and two tablets (named "Comparative Example 2") as shown in Table 4 below were administered. For MAD3 and MAD4, one tablet and two tablets of the enteric coated preparation of the previous Experimental Example 1 (Comparative Example 1) were administered, respectively.

**[Table 3]**

| Category | Group | Active ingredient | Formulation | Administration method |
|---|---|---|---|---|
| Multiple administration | MAD1 | 25 mg | Enteric coated capsule | After oral administration twice a day for 13 consecutive days, single administration on the morning of the 14th day. |
| | MAD2 | 50 mg | | |
| | MAD3 | 100 mg | | |
| | MAD4 | 200 mg | | |

**[Table 4]**

| Category | | Ingredient | Content (mg) |
|---|---|---|---|
| Premixing | Main ingredient | API | 27.80 |
| | Excipient | MCC(JRS-101) | 120.00 |
| | | Supertab 30GR | 120.00 |
| | Disintegrant | Crospovidone | 10.00 |
| | Lubricant | Mg Stearate | 3.00 |
| Post-mixing | Excipient | MCC(vivapur12) | 58.60 |
| | | Supertab 30GR | 58.60 |
| | Disintegrant | Crospovidone | 10.00 |
| | Binder | HPC-L | 10.00 |
| | Lubricant | Mg Stearate | 2.00 |
| Uncoated table | | | 420.00 |
| Coating | First coating agent | Opadry white 03B28796 | 8.00 |
| | Second coating agent | Acryl EZE 93O18508 | 34.00 |
| Coated tablet | | | 462.00 |

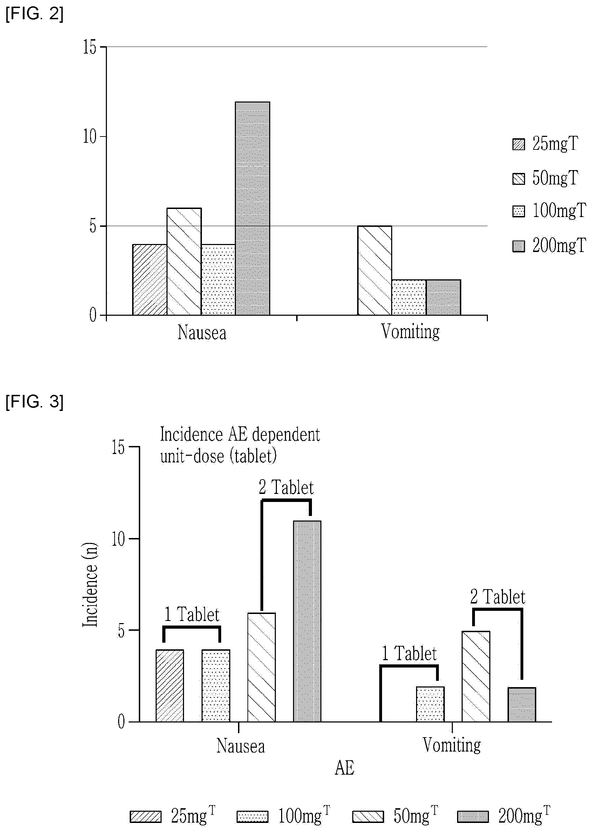

As a result, as can be seen in FIG. 2, it was confirmed that the side effects of nausea/vomiting have a low correlation with the administered dose of the drug, and rather, as shown in FIG. 3, they have a correlation between the number of tablets administered and the incidence of side effects. This is inferred to be because the surface area increases as the number of tablets increases, thereby affecting an increase in initial dissolution.

Based on the above clinical results, it could be expected that the side effects of nausea/vomiting were correlated with the initial dissolution and not the administered dose of the API. Accordingly, the following tablet to improve nausea/vomiting side effects was developed.

### Example 1

The active ingredient was prepared into a formulation through a dry granulation process, specifically as follows.

### (Step 1)

166.65 mg of active ingredient, 62.20 mg of microcrystalline cellulose (JRS-101), 62.15 mg of lactose hydrate (Supertab 30GR), 10.00 mg of povidone (PVP K90), and 3.00 mg of magnesium stearate were mixed.

### (Step 2)

The mixture of step 1 was prepared into a plate-shaped compressed product using a dry granulator, and pulverized using an oscillator to prepare a dry granulate.

### (Step 3)

57.00 mg of microcellulose (Vivapur12), 57.00 mg of lactose hydrate (Supertab 30GR), and 2.00 mg of magnesium stearate were added to the granule from step 2 and mixed.

### (Step 4)

The mixture of step 3 was compression molded to a total weight of 420.00 mg and produced into tablets (uncoated tablets). This was named '#1-1', and the content of ingredients contained in the tablet is the same as shown in #1-1 in Table 5 below.

Further, as shown in Table 5 below, #1-2 and #1-3 were prepared in the same manner as in #1-1 by changing each ingredient and content.

Each uncoated tablet produced above was subjected to a dissolution test under the following conditions according to the second dissolution method (paddle method) of the Korean Pharmacopoeia, and the results are shown in Table 5 below.
- Eluate: pH 6.8 buffer solution, 900 mL
- Rotation speed: 50 rpm
- Temperature: 37.0 ±0.5°C
- Dissolution sample collection time: 5 min, 10 min, 15 min, 30 min, 45 min, 60 min
- Analysis method: HPLC analysis method
- Detector: Ultraviolet absorptiometry (measurement wavelength: 260 nm)
- Column: C8 5 µm / 4.6 x 250 mm column
- Mobile phase: distilled water + methanol + trifluoroacetic acid

**[Table 5]**

| | | | #1-1 | #1-2 | #1-3 |
|---|---|---|---|---|---|
| Premixing | Main ingredient | API | 166.65 | 166.65 | 166.65 |
| | Excipient | MCC (JRS-101) | 62.20 | 62.20 | 62.20 |
| | | Supertab 30GR | 62.15 | 62.15 | 62.15 |
| | Binder¹⁾ | PVP K90 | 10.00 | - | - |
| | | HPC-L | - | 10.00 | - |
| | | PVP K30 | - | - | 10.00 |
| | Lubricant | Mg Stearate | 3.00 | 3.00 | 3.00 |
| Post-mixing | Excipient | MCC (vivapur12) | 57.00 | 57.00 | 57.00 |
| | | Supertab 30GR | 57.00 | 57.00 | 57.00 |
| | Disintegrant | Crospovidone | - | - | - |
| | | L-HPC (LH-11) | - | - | - |
| | | SSG | - | - | - |
| | | croscarmellose sod. | - | - | - |
| | Lubricant | Mg Stearate | 2.00 | 2.00 | 2.00 |
| Uncoated table (mg) | | | 420.00 | 420.00 | 420.00 |
| Uncoated table dissolution profile | | 5 min (%) | 57.50 | 57.62 | 72.80 |
| | | 10 min (%) | 83.24 | 89.03 | 89.32 |
| | | 15 min (%) | 88.48 | 93.65 | 91.67 |
| | | 30 min (%) | 91.73 | 95.90 | 93.16 |
| | | 45 min (%) | 93.39 | 96.87 | 94.63 |
| | | 60 min (%) | 94.65 | 97.91 | 95.36 |
| 1) the viscosity of each binder is as follows: | | | | | |
| - PVP K90: 300 to 700 mPa·s in 10% w/v aqueous solution at 20°C | | | | | |
| - HPC-L: 6 ~ 10 mPa·s in 2% w/v aqueous solution at 20°C | | | | | |
| - PVP K30: 5.5 ~ 8.5 mPa·s in 10% w/v aqueous solution at 20°C | | | | | |

As mentioned above, when only the type of binder was changed, the uncoated tablet dissolution rate within 5 minutes appeared to be within 75% for PVP K90, HPC-L, and PVP K30. Further, when PVP K90 and HPC-L were used as binders (#1-1, and #1-2), the initial release inhibition effect was higher than when PVP K30 was used (#1-3).

### Example 2

Following the results of Example 1, the dissolution rate due to the use of the disintegrant was evaluated. Specifically, an uncoated tablet was produced in the same manner as in Example 1, except that the ingredients used in the production of the uncoated tablet are changed as shown in Table 6 below, and the dissolution rate was evaluated.

**[Table 6]**

| | | | #2-1 | #2-2 | #2-3 | #2-4 | #2-5 | #2-6 |
|---|---|---|---|---|---|---|---|---|
| Premixing | Main ingredient | API | 166.65 | 166.65 | 166.65 | 166.65 | 166.65 | 166.65 |
| | Excipient | MCC (JRS-101) | 62.20 | 62.20 | 57.20 | 52.20 | 62.20 | 62.20 |
| | | Supertab 30GR | 62.15 | 62.15 | 57.15 | 52.15 | 62.15 | 62.15 |
| | Binder | PVP K90 | 10.00 | 10.00 | 20.00 | 30.00 | - | - |
| | | HPC-L | - | - | - | - | 10.00 | - |
| | | PVP K30 | - | - | - | - | - | 10.00 |
| | Lubricant | Mg Stearate | 3.00 | 3.00 | 3.00 | 3.00 | 3.00 | 3.00 |
| Post-mixing | Excipient | MCC (vivapur12) | 57.00 | 57.00 | 57.00 | 57.00 | 57.00 | 57.00 |
| | | Supertab 30GR | 57.00 | 57.00 | 57.00 | 57.00 | 57.00 | 57.00 |
| | Disintegrant | Crospovidone | 5.00 | 10.00 | 10.00 | 10.00 | 10.00 | 10.00 |
| | | L-HPC (LH-11) | - | - | - | - | - | - |
| | | SSG | - | - | - | - | - | - |
| | | croscarmellose sod. | - | - | - | - | - | - |
| | Lubricant | Mg Stearate | 2.0 | 2.0 | 2.0 | 2.0 | 2.0 | 2.0 |
| Uncoated table (mg) | | | 425 | 430.0 | 430.0 | 430 | 430.0 | 430.0 |
| Uncoated table dissolution profile | | 5 min (%) | 72.14 | 79.49 | 62.25 | 57.88 | 80.64 | 89.11 |
| | | 10 min (%) | 81.73 | 86.78 | 76.01 | 74.70 | 88.08 | 95.39 |
| | | 15 min (%) | 85.49 | 89.68 | 82.88 | 82.68 | 90.87 | 97.04 |
| | | 30 min (%) | 89.16 | 92.47 | 90.03 | 90.38 | 92.62 | 97.67 |
| | | 45 min (%) | 91.03 | 93.63 | 93.54 | 93.79 | 94.19 | 98.19 |
| | | 60 min (%) | 92.48 | 94.75 | 95.42 | 95.12 | 94.79 | 98.39 |

Comparing #2-1 with #2-2, the initial release inhibition effect decreases as the amount of disintegrant used increases, however, when the amount of binder used increases (#2-3, #2-4), the initial release inhibition effect can decrease again. Additionally, if the degree of binding of the binder is low when using a disintegrant (#2-5 and #2-6), the initial release inhibition effect is lowered, and therefore, the type and amount of disintegrant used must be adjusted in consideration of the binding force of the binder.

### Example 3

Following the results of Example 2, the dissolution rate was evaluated based on type and amount of disintegrant used. Specifically, an uncoated tablet was produced in the same manner as in Example 1, except that the ingredients used in the production of the uncoated tablet are changed as shown in Table 7 below, and the dissolution rate was evaluated.

**[Table 7]**

| | | | #3-1 | #3-2 | #3-3 | #3-4 | #3-5 |
|---|---|---|---|---|---|---|---|
| Premixing | Main | API | 166.65 | 166.65 | 166.65 | 166.65 | 166.65 |
| | ingredient | | | | | | |
| | Excipient | MCC (JRS-101) | 62.20 | 62.20 | 62.20 | 62.20 | 62.20 |
| | | Supertab 30GR | 62.15 | 62.15 | 62.15 | 62.15 | 62.15 |
| | Binder | PVP K90 | 10.00 | 10.00 | 10.00 | 10.00 | 10.00 |
| | | HPC-L | - | - | - | - | - |
| | | PVP K30 | - | - | - | - | - |
| | Lubricant | Mg Stearate | 3.00 | 3.00 | 3.00 | 3.00 | 3.00 |
| Post-mixing | Excipient | MCC (vivapur12) | 57.00 | 54.50 | 48.50 | 57.00 | 57.00 |
| | | Supertab 30GR | 57.00 | 54.50 | 48.50 | 57.00 | 57.00 |
| | Disintegrant | Crospovidone | - | - | - | - | - |
| | | L-HPC (LH-11) | 5.00 | 10.00 | 22.00 | - | - |
| | | SSG | - | - | - | 10.00 | - |
| | | croscarmellose sod. | - | - | - | - | 10.00 |
| | Lubricant | Mg Stearate | 2.00 | 2.00 | 2.00 | 2.00 | 2.00 |
| Uncoated table (mg) | | | 425.0 | 425.0 | 425.0 | 430.0 | 430.0 |
| Uncoated table dissolution profile | | 5 min (%) | 54.45 | 53.47 | 57.76 | 76.77 | 68.67 |
| | | 10 min (%) | 86.83 | 83.16 | 80.89 | 85.98 | 79.28 |
| | | 15 min (%) | 92.79 | 89.64 | 88.37 | 88.34 | 82.13 |
| | | 30 min (%) | 96.21 | 93.81 | 91.95 | 90.43 | 85.45 |
| | | 45 min (%) | 97.82 | 95.57 | 93.78 | 92.08 | 86.67 |
| | | 60 min (%) | 98.93 | 96.21 | 95.08 | 93.67 | 88.35 |

When the disintegrating effect of the disintegrant was low as in #3-1 to #3-3, the initial release inhibition effect was high even when the amount used was increased, however, when the disintegration effect of the disintegrant was high (#3-4, and #3-5), the initial release inhibition effect was slightly lower. Therefore, in order to increase the initial release inhibition effect, a disintegrant should not be used, a disintegrant should be used in an amount as small as possible, or a disintegrant with a low disintegration effect should be used.

### Example 4

In the previous Example 1, the initial release effect was evaluated based on the type of binder. In this Example 4, based on the results of Example 1, the effect of inhibiting the initial release was confirmed when the amount used during pre-mixing and post-mixing of excipients was adjusted. Specifically, an uncoated tablet was produced in the same manner as in Example 1, except that the ingredients used in the production of the uncoated tablet are changed as shown in Table 8 below, and the dissolution rate was evaluated.

**[Table 8]**

| | | | #4-1 | #4-2 | #4-3 | #4-4 |
|---|---|---|---|---|---|---|
| Premixing | Main ingredient | API | 166.65 | 166.65 | 166.65 | 166.65 |
| | Excipient | MCC (JRS-101) | 11.90 | 40.05 | 79.20 | 107.30 |
| | | Supertab 30GR | 11.85 | 40.00 | 79.15 | 107.25 |
| | Binder | PVP K90 | 10.00 | 10.00 | 10.00 | 10.00 |
| | | HPC-L | - | - | - | - |
| | | PVP K30 | - | - | - | - |
| | Lubricant | Mg Stearate | 3.00 | 3.00 | 3.00 | 3.00 |
| Post-mixing | Excipient | MCC (vivapur12) | 107.30 | 79.15 | 40.00 | 11.90 |
| | | Supertab 30GR | 107.30 | 79.15 | 40.00 | 11.90 |
| | Disintegrant | Crospovidone | - | - | - | - |
| | | L-HPC (LH-11) | - | - | - | - |
| | | SSG | - | - | - | - |
| | | croscarmellose sod. | - | - | - | - |
| | Lubricant | Mg Stearate | 2.00 | 2.00 | 2.00 | 2.00 |
| Uncoated table (mg) | | | 420.0 | 420.0 | 420.0 | 420.0 |
| Uncoated table dissolution profile | | 5 min (%) | 70.08 | 72.30 | 36.57 | 41.17 |
| | | 10 min (%) | 86.87 | 88.25 | 73.64 | 79.43 |
| | | 15 min (%) | 90.79 | 94.28 | 82.56 | 93.32 |
| | | 30 min (%) | 93.69 | 97.50 | 87.95 | 96.74 |
| | | 45 min (%) | 95.84 | 99.96 | 90.86 | 98.04 |
| | | 60 min (%) | 97.18 | 101.51 | 93.04 | 98.61 |

As mentioned above, even if the total amount of excipients used is the same, when more excipients were used in pre-mixing (#4-3, and #4-4), the initial release inhibition effect was higher than when more excipients were used in post-mixing (#4-1, and #4-2).

However, when the ratio of the pre-mixed part and the post-mixed part was biased to one side, it was confirmed that the uncoated tablet mass was not constant as the tableting progresses. To evaluate this, a formulation uniformity test was conducted. The formulation uniformity test was conducted as follows, and the results are summarized in Table 9.
- Test method for formulation uniformity: 1 tablet of the preparation was taken, placed in a 50 mL volumetric flask, 30 mL of diluent was added thereto, and the mixture was stirred until the tablet was completely disintegrated, and then ultrasonically extracted for 15 minutes or more, and then the mark was matched with the diluent. An appropriate amount of this solution was centrifuged at 3000 rpm for 10 minutes, then exactly 7 mL of the supernatant was taken, placed in a 200 mL volumetric flask, and the diluent was added to match with the mark. An appropriate amount of this solution was taken, filtered through a 0.45 µm membrane filter, the first 2 mL was discarded, and the filtrate was used as the sample solution.
- Diluent: water + methanol
- Analysis method: HPLC method
- Detector: Ultraviolet absorptiometry (measurement wavelength: 260 nm)
- Column: C8 5 µm / 4.6x250 mm column
- Mobile phase: distilled water + methanol + trifluoroacetic acid

**[Table 9]**

| | #4-1 | #4-2 | #4-3 | #4-4 | #1-1 |
|---|---|---|---|---|---|
| 1 | 90.17 | 106.27 | 104.75 | 84.76 | 100.88 |
| 2 | 100.26 | 104.36 | 103.84 | 84.86 | 100.46 |
| 3 | 89.95 | 103.28 | 102.81 | 107.99 | 102.58 |
| 4 | 99.43 | 100.28 | 103.19 | 108.33 | 99.90 |
| 5 | 109.50 | 99.89 | 101.96 | 102.57 | 99.31 |
| 6 | 85.62 | 98.93 | 101.77 | 96.41 | 99.81 |
| 7 | 114.74 | 98.56 | 101.29 | 85.18 | 100.92 |
| 8 | 109.66 | 99.14 | 101.75 | 96.34 | 101.63 |
| 9 | 100.24 | 102.46 | 101.32 | 103.95 | 100.89 |
| 10 | 108.74 | 102.97 | 101.15 | 104.14 | 100.77 |
| Average | 100.83 | 101.61 | 102.38 | 97.45 | 100.72 |
| SD | 9.86 | 2.62 | 1.22 | 9.52 | 0.94 |
| RSD | 9.78 | 2.58 | 1.19 | 9.77 | 0.93 |
| Judgment value (L) | 23.55 | 6.27 | 3.28 | 24.26 | 2.25 |

It was confirmed that when the excipient ratios of the pre-mixed part and the post-mixed part were used similarly as mentioned above, the formulation uniformity was excellent. When considered together with the effect of inhibiting initial release, the excipient ratio of the pre-mixed part and the post-mixed part are similar, but it is judged that when the excipient ratio of the pre-mixed part is slightly higher (#1-1, #4-3), it has an initial release inhibiting effect and it will be possible to produce tablets with equal quality.

### Example 5

When the results of Examples 1 to 4 are combined, #1-1 uncoated tablet, in which the viscosity of the binder was high, the disintegrant was not soluble, the excipients were used in similar amounts in pre-mixing and post-mixing, but slightly more excipients were used in the premixing, showed the most optimal results. In this Example 5, based on this, a coated tablet was produced by applying a coating agent to an uncoated tablet, and the dissolution profile of the coated tablets was evaluated.

Specifically, the uncoated tablet #1-1 produced in Example 1-1 was primarily coated with Opadry white 03B28796 coating agent, and then secondarily coated with Acryl EZE 93O18508, which is an enteric coating agent. The amount of coating base was applied as shown in Table 10 below. In order to check the acid resistance and dissolution rate of each produced coated tablet, tests were performed according to the following methods and conditions.
- Dissolution method: second dissolution method (paddle method) of the Korean Pharmacopoeia
- Eluate: buffer transition (pH1.2 → pH6.8)
- Eluent volume: 900 → 1000 mL
- Elutriator temperature: 37.5°C ± 0.5°C
- Paddle speed: 100 rpm
- Analysis method: HPLC method
- Detector: Ultraviolet absorptiometry (measurement wavelength: 260 nm)
- Column: C8 5 µm / 4.6x250 mm column
- Mobile phase: distilled water + methanol + trifluoroacetic acid

**[Table 10]**

| | | #5-1 | #5-2 | #5-3 | #5-4 | #5-5 | #5-6 | #5-7 |
|---|---|---|---|---|---|---|---|---|
| First coating agent (mg) | | 8.0 | - | 8.00 | 8.00 | 8.00 | 8.00 | 8.00 |
| Second coating agent (mg) | | 34.0 | 34.00 | - | 17.00 | 25.50 | 42.50 | 51.00 |
| Coated table dissolution profile | pH 1.2, 2 hr (%) | 0 | 0 | 97.5 | 0 | 0 | 0 | 0 |
| | pH 6.8, 5 min (%) | 0 | 0.10 | 99.20 | 3.10 | 0.20 | 0 | 0 |
| | pH 6.8, 10 min (%) | 6.3 | 23.00 | 99.20 | 60.30 | 15.00 | 5.90 | 1.20 |
| | pH 6.8, 15 min (%) | 47.2 | 67.90 | 99.20 | 94.30 | 58.20 | 37.90 | 19.60 |
| | pH 6.8, 30 min (%) | 97.5 | 100.00 | 99.20 | 101.20 | 96.10 | 97.70 | 94.70 |
| | pH 6.8, 45 min (%) | 99.6 | 100.50 | 99.10 | 101.30 | 100.70 | 102.30 | 99.40 |
| | pH 6.8, 60 min (%) | 99.7 | 102.00 | 99.00 | 101.40 | 101.70 | 102.70 | 99.50 |

When checking the dissolution results based on the progress of the primary coating (#5-1, #5-2), it was confirmed that as the secondary coating of enteric coating was carried out in the same manner, acid resistance was secured in both conditions, but as compared to #5-1, which was subjected to primary coating, dissolution of #5-2 progresses more rapidly under the condition of pH 6.8. It could be confirmed that #5-3, which was not subjected to secondary coating, was unable to secure acid resistance as expected. The primary coating was carried out in the same manner, and as a result of checking the dissolution rate based on the secondary coating amount, when coating was carried out at 4% to 12% of 100 parts by weight of uncoated tablets, acid resistance was secured in all cases, but in the case of #5-4, which was coated at 4% compared to 100 parts by weight of the uncoated tablet, some tablets were found to have swollen due to retaining moisture in the eluator. From the results, it was confirmed that acid resistance was secured, but the coating amount of #5-4 was not sufficient as the tablet shape was not maintained in acidic conditions. When the secondary coating base was used at 6% to 12% of 100 parts by weight of the uncoated tablet, acid resistance was secured, and no change in properties occurred after dissolution in acidic conditions.

### Example 6

In order to confirm the difference in the initial dissolution rate between Comparative Examples 1 and 2, in which nausea/vomiting was previously confirmed as a side effect in Experimental Examples 1 and 2, and #5-1 coated tablet produced in Example 5, comparative dissolution was confirmed under three conditions as follows, and the results are shown in Tables 11 to 13, respectively.
1) Simple comparative dissolution: for one tablet each of Comparative Example 1, Comparative Example 2, and #5-1 formulation, the dissolution test was conducted in the same manner as in Example 1, and the results are shown in Table 11.
2) Comparative dissolution at the same dose: 2 tablets of Comparative Example 1 and 1 tablet of Comparative Example 2 were evaluated together, and one tablet of formulation #5-1 was evaluated, and the dissolution test was conducted in the same manner as in Example 1. The results are shown in Table 12 below.
3) Comparative dissolution under pH conditions in the body: For one tablet each of Comparative Example 1, Comparative Example 2, and #5-1 formulation, the dissolution test was conducted in the same manner as in Example 5. The results are shown in Table 13 below.

**[Table 11]**

| Time(min) | 5 | 10 | 15 | 30 | 45 | 60 | 90 | 120 |
|---|---|---|---|---|---|---|---|---|
| Comparative Example 1 | 0.00 | 24.51 | 75.66 | 89.01 | 94.59 | 96.14 | 97.81 | 98.84 |
| Comparative Example 2 | 0.00 | 16.10 | 52.38 | 88.39 | 95.84 | 97.70 | 99.77 | 101.47 |
| #5-1 | 0.00 | 0.85 | 19.36 | 91.40 | 100.59 | 101.12 | 101.54 | 102.12 |

**[Table 12]**

| Time(min) | 5 | 10 | 15 | 30 | 45 | 60 | 90 | 120 |
|---|---|---|---|---|---|---|---|---|
| Comparative Example 1 (2 tablet)+ Comparative Example 2(1 tablet) | 0.00 | 23.50 | 65.32 | 89.80 | 91.75 | 92.70 | 93.64 | 94.20 |
| #5-1 (1 tablet) | 0.00 | 4.55 | 27.86 | 89.96 | 92.90 | 94.58 | 95.26 | 95.79 |

**[Table 13]**

| pH/Time(min) | pH1.2 | | pH6.8 | | | | | |
|---|---|---|---|---|---|---|---|---|
| | 0 | 120 | 125 | 130 | 135 | 150 | 165 | 180 |
| Comparative Example 1 | 0.00 | 0.00 | 0.00 | 59.31 | 97.04 | 99.01 | 99.40 | 99.28 |
| Comparative Example 2 | 0.00 | 0.00 | 0.09 | 64.35 | 96.56 | 100.36 | 100.51 | 100.29 |
| #5-1 | 0.00 | 0.00 | 0.00 | 5.37 | 38.79 | 199.83 | 1101.74 | 102.06 |

As mentioned above, it was confirmed that the #5-1 formulation according to the present invention is controlled in initial release when compared with Comparative Examples 1 and 2.

### Experimental Example 3

In order to confirm the effect of improving the side effect of the #5-1 formulation produced as a coated tablet as above, a clinical phase 1 was conducted in healthy adult subjects.

### Experimental Example 3-1

Clinical testing on two phase 1 single-dose cases was designed in open level, two-part, fixed-sequence, three-period manner in 24 and 12 healthy adult subjects, respectively. Single administration was performed using tablet #5-1. As a control group, a clinical testing on one phase 1 single-dose case was designed in a randomized, open-label, and cross-over manner in 36 healthy adults, and a clinical testing was conducted in which Comparative Example 1 was administered as a single dose of 2 tablets. Table 14 below shows the comparative results of the incidence of nausea/vomiting side effects based on the relevant clinical results.

**[Table 14]**

| Formulation | Comparative Example 1 | #5-1 | #5-1 |
|---|---|---|---|
| Dosage (mg) | 200 | 150 | 150 |
| Administration day (day) | 1 | 1 | 1 |
| Target number of people | 36 | 24 | 12 |
| (person) | | | |
| Nausea incidence | 11% | 0% | 0% |
| Vomiting incidence | 6% | 0% | 0% |

### Experimental Example 3-2

Using the #5-1 formulation produced as a coated tablet above, clinical testing on two phase 1 single-dose cases was designed in open level, two-part, fixed-sequence, three-period manner in 48 and 12 healthy adult subjects, respectively. Multiple administration was performed using formulation #5-1. In Table 15 below, the incidence of nausea/vomiting side effects due to multiple administration of Comparative Example 1, which was confirmed in Experimental Example 1, was compared with the results of two multiple-dose clinical trials of formulation #5-1. In each clinical testing, the administered dose was administered twice a day.

**[Table 15]**

| Formulation | Comparative Example 1 | Comparative Example 1 | #5-1 | #5-1 |
|---|---|---|---|---|
| Dosage (mg) | 100 | 200 | 150 | 150 |
| Administration day (day) | 3 | 3 | 3 | 3 |
| Target number of people (person) | 6 | 6 | 48 | 12 |
| Nausea | 17% | 33% | 15% | 0% |
| Vomiting | 17% | 0% | 0% | 0% |

As mentioned above, it was confirmed that when administering the #5-1 formulation, the incidence of nausea/vomiting side effects was significantly improved compared to the existing formulation.

## Claims

1. A controlled release pharmaceutical composition comprising: a compound represented by the following Chemical Formula 1, or a pharmaceutically acceptable salt thereof; a binder; and one or more pharmaceutically acceptable excipients,
wherein the pharmaceutical composition shows a dissolution rate of 75% or less within 5 minutes in a pH 6.8 solution at 37°C based on an uncoated tablet.

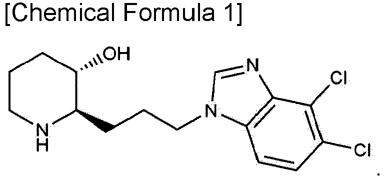

2. The controlled release pharmaceutical composition of claim 1, wherein:
the controlled release pharmaceutical composition shows a dissolution rate of 65% or less within 5 minutes in a pH 6.8 solution at 37°C based on an uncoated tablet.

3. The controlled release pharmaceutical composition of claim 1, wherein:
the controlled release pharmaceutical composition shows a dissolution rate of 90% or more within 30 minutes in a pH 6.8 solution at 37°C based on an uncoated tablet.

4. The controlled release pharmaceutical composition of claim 1, wherein:
the controlled release pharmaceutical composition is a coated tablet containing a coating agent, and shows a dissolution rate of 20% or less within 10 minutes in a pH 6.8 solution at 37°C based on the coated tablet.

5. The controlled release pharmaceutical composition of claim 4, wherein:
the controlled release pharmaceutical composition shows a dissolution rate of less than 60% within 15 minutes in a pH 6.8 solution at 37°C based on the coated tablet.

6. The controlled release pharmaceutical composition of claim 4, wherein:
dissolution does not occur in a pH 1.2 solution at 37°C based on the coated tablet.

7. The controlled release pharmaceutical composition of claim 4, wherein:
the coated tablet is an enteric coated tablet.

8. The controlled release pharmaceutical composition of claim 4, wherein:
the coating agent is contained in an amount of 6 to 12 parts by weight relative to 100 parts by weight of the uncoated tablet.

9. The controlled release pharmaceutical composition of claim 1, wherein:
the binder is contained in an amount of 1 to 20 parts by weight relative to 100 parts by weight of the uncoated tablet.

10. The controlled release pharmaceutical composition of claim 1, wherein:
the binder is polyvinylpyrrolidone, hydroxypropyl cellulose, carboxymethyl cellulose sodium, carboxymethyl cellulose calcium, hydroxyethyl cellulose, hydroxypropyl methylcellulose, gelatin, gum arabic, or xanthan gum.

11. The controlled release pharmaceutical composition of claim 10, wherein:
a viscosity of the binder is 5 to 1000 mPa·s in a 10% w/v aqueous solution at 20°C.

12. The controlled release pharmaceutical composition of claim 10, wherein:
the viscosity of the binder is 5 to 500 mPa·s in a 2% w/v aqueous solution at 20°C.

13. The controlled release pharmaceutical composition of claim 1,
which comprises 0 to 10 parts by weight of a disintegrant relative to 100 parts by weight of the uncoated tablet.

14. The controlled release pharmaceutical composition of claim 13, wherein:
the disintegrant is a super disintegrant, and the disintegrant is contained in an amount of 50 parts by weight or less relative to 100 parts by weight of the binder.

15. The controlled release pharmaceutical composition of claim 14, wherein:
the super disintegrant is sodium starch glycolate, croscarmellose sodium, or crospovidone.

16. The controlled release pharmaceutical composition of claim 1, wherein:
the pharmaceutical composition comprises (i) a pre-mixed granule containing a compound represented by Chemical Formula 1, or a pharmaceutically acceptable salt thereof; a binder; and an excipient, and (ii) a post-mixed material containing an excipient.

17. The controlled release pharmaceutical composition of claim 16, wherein:
a weight ratio of the excipient contained in the pre-mixed granule and the excipient contained in the post-mixed material is 4:6 to 6:4.
